# EUROPÄISCHE PATENTANMELDUNG

(11) **EP 2 679 276 A1**
(43) Veröffentlichungstag der Anmeldung: **01.01.2014**
(21) Anmeldenummer: 12004849.1
(22) Anmeldetag: 28.06.2012
(51) Int. Cl.: A61N 1/05

(54) **Epikardiales Stimulationsband**

(71) Anmelder: Peter Osypka Stiftung, 79639 Grenzach-Wyhlen (DE)
(72) Erfinder: Osypka, Peter, Dr., D-79639 Grenzach-Wyhlen (DE)

(57) **Zusammenfassung**

Implantierbares epikardiales Stimulationsband, auf dessen Oberfläche sich Areale von Elektroden befinden, die Teile des Herzens beim Menschen, insbesondere die Vorhöfe, nach einer Herz Operation in Verbindung mit einem außerhalb des Körpers anschließbaren Herzschritimacher oder Defibrillator überwachen und/oder stimulieren und nach erfolgter Anwendung jederzeit leicht durch Zug entfembar ist.

## Beschreibung

### Die Erfindung

Die Erfindung betrifft ein implantierbares epikardiales Stimulationsband, auf dessen Oberfläche sich Areale von Elektroden befinden, die Teile des Herzens beim Menschen, insbesondere die Vorhöfe, nach einer Herz Operation in Verbindung mit einem außerhalb des Körpers anschließbaren Herzschrittmacher oder Defibrillator überwachen und/oder stimulieren und nach erfolgter Anwendung jederzeit leicht durch Zug entfernbar ist.

### Problem

Temporäre Myokardiale Elektroden (auch als Herzdrähte bekannt), ermöglichen nach einer Herzoperationen eine externe Stimulation des Herzens. Derartige Elektroden sind seit vielen Jahren bekannt und werden routinemäßig nach jeder offenen Herzoperation zur Stimulation der Vorhöfe und Ventrikel eingesetzt. Die Fixierung und damit die Stimulation der Vorhöfe des Herzens, insbesondere des linken Vorhofes mit Herzdrähten, bereiten wegen seiner speziellen anatomischen Lage erhebliche Schwierigkeiten. Vom linken Vorhof ist bei einer offenen Herz Operation höchstens das linke Herzohr sichtbar, das wegen seiner fragilen Gewebestruktur für eine Fixierung von Herzdrähten nicht geeignet ist Der eigentliche linke Vorhof befindet sich auf der Rückseite des Herzens und ist für eine temporäre Elektroden Fixierung schwer zugänglich. Daher wird auf die Stimulation des linken Vorhofs, trotz seiner großen medizinischen Bedeutung für die Prophylaxe bzw. Therapie des Vorhofflimmems, oft verzichtet.

### Lösungsweg

Es besteht daher die Aufgabe eine Elektroden Anordnung der eingangs genannten Art zu schaffen, die es ermöglicht, einfacher und schneller die Vorhöfe des Herzens getrennt oder gemeinsam zu stimulieren bzw. zu kardiovertieren (deffibrillieren).
Zur Lösung dieser Aufgabe ist es vorgesehen, dass die notwendige Stimulation/Kardioversion mit einer Anordnung, bestehend aus einem implantierbarem flexiblen Stimutationsband, auf dessen dem Herzen zugewandten Oberfläche, sich mindestens ein elektrischer Pol befindet, vorzugsweise sich Areale von Elektroden Polen befinden, die es ermöglichen, Teile des Herzens, vorzugsweise sowohl den linken als auch den rechten Vorhof nach einer Herz Operation in Verbindung mit einem außerhalb des Körpers anschließbaren Herzschrittmacher oder Defibrillator zu überwachen und/oder zu stimulieren und das nach erfolgter Anwendung jederzeit leicht durch Zug entfembar ist.
Um die Vorhöfe des Herzens physisch leicht zu erreichen, ist das Stimulationsband so aufgebaut, dass es sich leicht nach der Herz Operation, unter die Vorhöfe des Herzens platzieren lässt, sodass ein guter Kontakt für die elektrische Stimulation erreicht wird.

### Vorteile

Der große Vorteil der Erfindung liegt darin, dass mit Hilfe eines epikardialen Stimulationsbandes, eine einfache Platzierung unter den Vorhöfen des Herzens vorgenommen werden kann und damit nicht nur eine Stimulation, sondem auch, falls Vorhofflimmem auftritt, dieses mit geringer Energie zu kardiovertieren.

### Zeichnungen

Weitere Einzelheiten, Merkmale und Vorteile der Erfindung lassen sich dem folgenden Beschreibungsteil entnehmen, in dem anhand der Zeichnungen und Ausführungsbeispiele die Erfindung näher erläutert wird. Sie zeigen in schematischer Darstellung in
Fig.1 Eine beispielhafte Anordnung des Epikardialen Stimulationsbandes über dem linken und rechten Vorhof auf der Rückseite des Herzens
Fig.2 Eine weitere beispielhafte Anordnung eines aus zwei Teilen bestehenden Epikardialen Stimulationsbandes über dem linken und rechten Vorhof auf der Rückseite des Herzens.
Fig.3 Eine weitere beispielhafte Anordnung des Epikardialen Stimulationsbandes über dem linken und rechten Vorhof auf der Rückseite des Herzens
Fig.4 Eine detaillierte Darstellung des Epikardialen Stimulationsbandes
Fig.5-1 bis 5-5 Weitere detaillierte Darstellungen des Epikardialen Stimulationsbandes
Fig.6 Eine detaillierte Darstellung des Epikardialen Stimulationsbandes mit einer Aufnahme für ein mechanisches Hilfswerkzeug zur Einführung des Bandes unter das Herz.
Fig.7 Eine beispielhafte Anordnung des Epikardialen Stimulationsbandes über dem linken und rechten Vorhof auf der Vorderseite des Herzens
Fig.8 Eine beispielhafte Anordnung des Epikardialen Stimulationsbandes mit auf einem Kunststoffband aufgebrachten Polen.

### Beschreibung der Abbildung

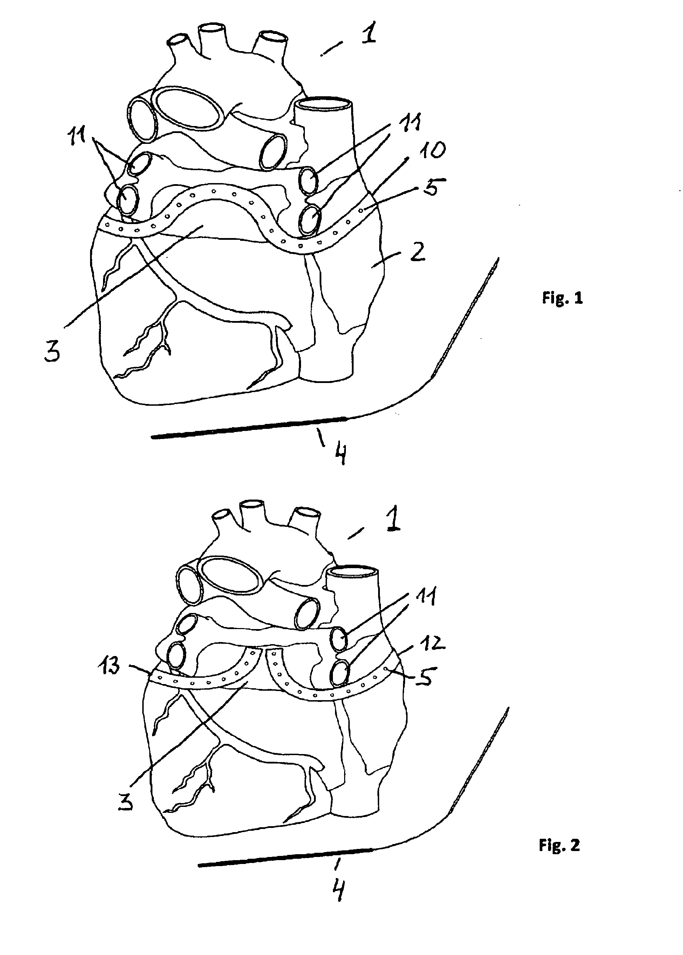

Fig.1 Zeigt eine beispielhafte Anordnung des Epikardialen Stimulationsbandes (10) über dem linken(3) und rechten Vorhof(2) auf der Rückseite des Herzens(1) Man erkennt die geschwungene Form des Bandes(10), die durch ein in dem Band integrierten vorgeformten Nitinol Draht erfolgt. Das Band kann zunächst in gestraffter Form unter das Herz bis zu den Pulmonal Venen (11) geführt werden. Danach erfolgt durch Nachlassen der Zugkraft am Band durch den vorgeformten Nitinol Draht automatisch die geschwungene Form unter dem linken Vorhof (3). Das Band(10) überdeckt dabei sowohl den linken als auch den rechten Vorhof(2). Auf der dem Herzen zugewandten Seite des Bandes(10) befinden sich in entsprechender Position Pole(5), die in Verbindung mit einem externen Schrittmacher die Vorhöfe elektrisch stimulieren können. Die Stimulation kann sowohl zwischen den einzelnen Polen des Bandes erfolgen, als auch zwischen einem Pol auf dem Band gegen einen indifferenten Pol (4) außerhalb des Herzens Die dem Herzen abgewandte Seite des Stimulation Bandes ist durch eine dünne flexible Kunststoffschicht elektrisch isoliert.

Fig.2 Zeigt eine weitere beispielhafte Anordnung eines aus zwei Teilen(12)(13) bestehenden Stimulation Bandes Ober dem linken(3) und rechten Vorhof(12) auf der Rückseite des Herzens. Die entsprechende Form des Bandes wird mit unterschiedlich vorgebogenen Nitinol Drähten erreicht.

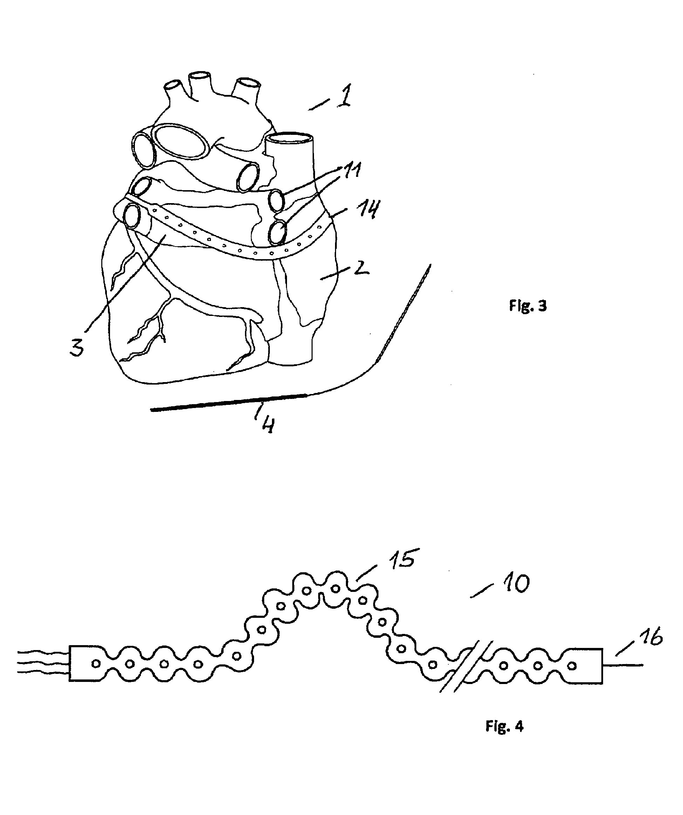

Fig.3 Zeigt eine weitere beispielhafte Anordnung des Stimulation Bandes(14) über dem linken und rechten Vorhof auf der Rückseite des Herzens.

Das Stimulationsband besteht aus einem flexiblen Material. Geeignete Materialien sind für alle Ausführungsformen beispielsweise Silikon, Polyurethan, Polyester, Seide und dergleichen. Auch eine Folie aus biokompatiblem Kunststoff wie z.B. Polyimid, ist geeignet.

Das Stimulationsband kann unterschiedliche Strukturen aufweisen. So kann das Band beispielsweise ein Geflecht, ein Gewebe oder eine Folie sein.

Die Form des Stimulationsbandes lässt sich an die Anatomie des Herzens anpassen. Die Formgebung wird durch einen aus einem Material mit Formgedächtnis bestehenden Draht, vorzugsweise Nitinol, unterstützt.

Das Stimulationsband hat zwei Seiten. Die dem Herzen zugewandte Seite trägt mindestens einen Pol. Die dem Herzen abgewandte Seite muss elektrisch isoliert sein. Das geschieht beispielsweise durch eine dünne flexible Kunststoffschicht. Die isolierende Schicht kann beispielsweise aus Silikon, Polyurethan, Parylene. Polyimid oder Nanomaterial bestehen.

Das Stimulationsband trägt mindestens einen Pol. In einer bevorzugten Ausführungsform trägt das Stimulationsband mehrere Pole, die unterschiedlich geformt sein können. Die Pole können beispielsweise als Halbkugel, Draht, Litze, Wendel, in Zig-Zag Form, als Netz, Geflecht, Gitter oder als elektrisch leitendes Band ausgebildet sein.

Die Anordnung der Pole auf dem Band ist variabel, sodass eine Anordnung entsprechend der anatomischen Lage der Vorhöfe möglich ist.

Geeignete Materialien für die Pole sind beispielsweise Edelstahl, MP35 N, Gold, Platin, Karbon, Nitinol und dergleichen. Auch ein leitfähiger mit Nanopartikeln gefüllter biokompatibler Kunststoff ist geeignet.

Die Fixierung der Pole auf dem Stimulationsband kann durch Kleben, Flechten, Weben, Ultraschall Schweißen, Nähen, Löten oder Laser verschmelzen erfolgen.

Wie oben ausgeführt, unterliegt die Ausgestaltung der Pole einer großen Varianz (mehrere Materialien, eine Vielzahl von Formen). Es ist daher vorstellbar, dass die Gestaltung der Pole auf dem epikardialen Stimulationsband als Kombination der oben genannten Eigenschaften in unterschiedlicher Form ausgeführt werden kann.

Fig.4 Zeigt eine detaillierte Darstellung des Stimulation Bandes(10) in der dem Herzen zugewandten Seite. Zur besseren Formgestaltung des Bandes ist es in der Länge teilweise oder ganz wellenförmig(15) ausgeführt. Innerhalb des isolierten Bandes ist ein geformter Nitinoldraht(16) integriert.

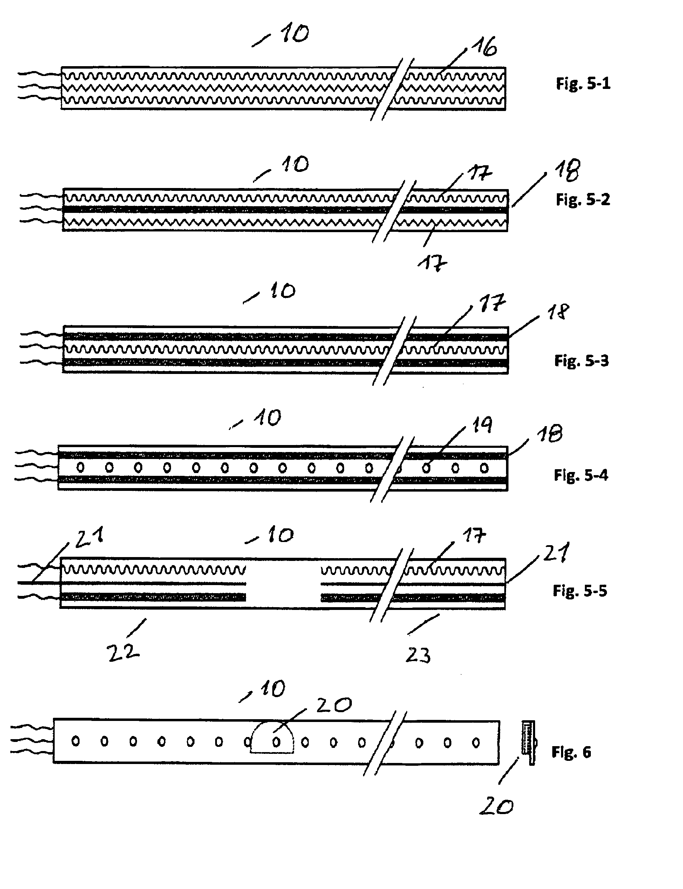

Fig.5.1 bis 5.5 Zeigen weitere detaillierte Darstellungen des Stimulation Bandes(10) in der dem Herzen zugewandten Seite.
5.1 Zeigt drei Pole als wellenförmige Drähte(16) ausgebildet.
5.2 Zeigt zwei außenliegende wellenförmige Draht Pole(17) mit einem in der Mitte des Bandes liegenden Pol in Form einer Wendel(1 8).
5.3 Zeigt zwei außenliegende Wendeln (18) mit einem in der Mitte des Bandes liegenden wellenförmigen Draht(17).
5.4 Zeigt zwei außenliegende Wendeln (18) mit in der Mitte des Bandes liegenden Polkappen(19).
5.5 Zeigt einen außenliegenden wellenförmigen Draht Pol (17) mit einem in der Mitte des Bandes liegenden Pol in Form eines Drahtes(21) und einem in dem unteren Teil des Bandes liegenden Pol in Form einer Wendet(18). Die Band Elektrode ist in zwei Pol Bereiche, jeweils in den linken(22) und rechten Vorhof(23) aufgeteilt. Es ist daher leicht vorstellbar, dass die Gestaltung der Pole auf dem Epikardialen Stimulations Band als Kombination der oben genannten Eigenschaften in unterschiedlichster Form ausgeführt werden kann.

Fig.6 Zeigt eine detaillierte Darstellung des Stimulationsbandes(10) mit einer Aufnahme(20) für ein mechanisches Hilfswerkzeug (z.B. Kunststoff Spatel) oder Magnet zum Einführen des Stimulation Bandes unter das Herz.

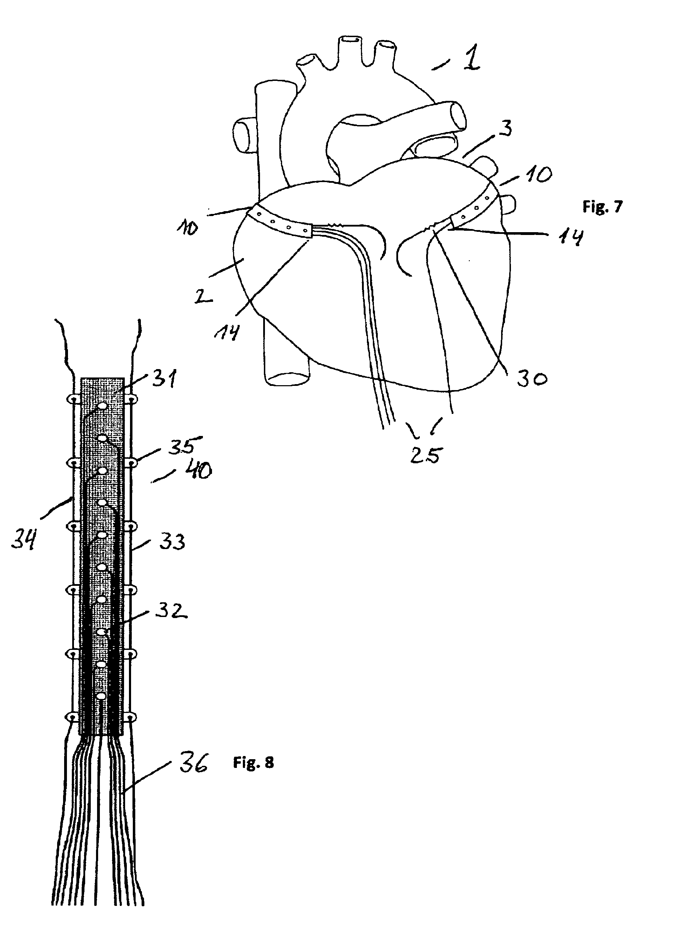

Fig.7 Zeigt eine beispielhafte Anordnung des Epikardialen Stimulationsbandes (10) über dem linken(3) und rechten Vorhof(2) auf der Vorderseite des Herzens(1). Die beiden Enden des Bandes(14) werden auf der Vorderseite der Ventrikel mit der üblichen Fixierungs Art (hier Zjg-Zag)(30), wie bei den üblichen Herzdrähten, temporär befestigt. Die Zuleitungen der Elektroden(25) münden in einer üblichen Öffnung im Brustkorb, durch die sie später durch einfachen Zug entfernt werden.

Fig.8 Zeigt eine beispielhafte Anordnung des Epikardialen Stimulationsbandes (40) mit auf einem Kunststoffband(31) aufgebrachten Polen(32). Dieses Stimulationsband eignet sich besonders zur Überwachung und Stimulation des linken Ventrikels und wird zusammen mit den elektrischen Zuleitungen (36) aus Kunststoff Fasern (z.B. Polyester) gewebt. Die Elektroden (32) sind Metallplättchen, die auf dem Kunststoffband (31) z.B. mittels Ultraschall aufgeschweißt werden. Die Fixierung der Stimulationsbandes(30) auf dem Herzen erfolgt durch hindurch ziehen der Fäden (33)(34) durch das Myokard und den Öffnungen (35) in dem Band(31). Zum Entfernen des Stimulationsbandes werden von außen die Fäden (33)(34)gezogen. wodurch sich das Band vom Herzen löst.

## Patentansprüche

1. Elektrodenanordnung zur Überwachung und Stimulation des Herzens nach einer Herz Operation umfassend:
ein flexibles, geformtes Stimulationsband(10), das auf der Herz abgewandten Seite isoliert ist und auf der Herz zugewandten Seite mit mindestens einem Pol oder mehr als einen Pol (5) bestückt ist;
eine indifferente Elektrode(4), die außerhalb des Herzens positioniert werden kann.

2. Elektrodenanordnung zur Überwachung und Stimulation des Herzens nach einer Herz Operation nach Anspruch 1 **dadurch gekennzeichnet,**
**dass** ein flexibles, geformtes Stimulationsband(10), das auf der Herz abgewandten Seite isoliert ist und auf der Herz zugewandten Seite mit mindestens einem elektrischen Pol oder mehr als einen Pol (5) bestückt ist, sich über den linken und rechten Vorhof erstreckt.

3. Elektrodenanordnung nach Anspruch 1 und 2 **dadurch gekennzeichnet,**
**dass** das Stimulationsband aus Silikon, Dacron ,Polyurethan, Polyester, Seide oder einer Folie aus biokompabilen Kunststoff besteht.

4. Elektrodenanordnung nach Anspruch 1 bis 3 **dadurch gekennzeichnet,**
**dass** die Struktur des Stimulationsbandes als Geflecht, Gewebe oder Folie ausgebildet sein kann.

5. Elektrodenanordnung nach Anspruch 1 bis 4 **dadurch gekennzeichnet,**
**dass** die Form des Stimulationsbandes entsprechend der Anatomie des Herzens geformt ist und durch einen aus einem Material mit Formgedächtnis bestehenden Draht, vorzugshalber Nitinol, in der Formgebung unterstützt wird.

6. Elektrodenanordnung nach Anspruch 1 bis 5 **dadurch gekennzeichnet,**
**dass** die Isolierung durch Beschichtung der Herz abgewandten Seite des Stimulationsbandes mit Silikon, Polyurethan, Parylene, Polyimid oder Nanomaterial erfolgen kann.

7. Elektrodenanordnung nach Anspruch 1 bis 6 **dadurch gekennzeichnet,**
**dass** das Material der Pole aus Edelstahl, MP35 N, Gold, Platin, Karbon, Nitinol, oder leitfähigem mit Nano Partikeln gefüllten Kunststoff Material besteht.

8. Elektrodenanordnung nach Anspruch 1 bis 7 **dadurch gekennzeichnet,**
**dass** die Pole als Halbkugel, Draht, Litze, Wendel, in Zig-Zag Form, als Netz, Geflecht, Gitter, oder als elektrisch leitendes Band ausgebildet sind.

9. Elektrodenanordnung nach Anspruch 1 bis 8 **dadurch gekennzeichnet,**
**dass** die Pole auf dem Band je nach der anatomischen Lage der Vorhöfe entsprechend angeordnet sind.

10. Elektrodenanordnung nach Anspruch 1bis 9 **dadurch gekennzeichnet,**
**dass** die Fixierung der Pole auf dem Band durch Kleben, Flechten, Weben, Ultraschall schweißen, Löten, Nähen oder LASER verschmelzen erfolgt.

11. Elektrodenanordnung nach Anspruch 1bis 10 **dadurch gekennzeichnet,**
**dass** die Positionierung des Bandes mit einem Hilfswerkzeug z.B. Spatel oder Magneten unterstützt wird.
